**Europäisches Patentamt**

**European Patent Office**

**Office européen des brevets**

(19)

(11) Veröffentlichungsnummer: **0 016 405**
**B1**

(12) # EUROPÄISCHE PATENTSCHRIFT

(45) Veröffentlichungstag der Patentschrift:
**25.11.81**

(21) Anmeldenummer: **80101258.4**

(22) Anmeldetag: **12.03.80**

(51) Int. Cl.³: **C 07 C 11/02**, C 07 C 1/20 //
B 01 J 29/04

(54) **Herstellung von Olefinen aus Rohmethanol.**

(30) Priorität: **14.03.79 DE 2909928**

(43) Veröffentlichungstag der Anmeldung:
**01.10.80 Patentblatt 80/20**

(45) Bekanntmachung des Hinweises auf die Patenterteilung:
**25.11.81 Patentblatt 81/47**

(84) Benannte Vertragsstaaten:
**AT BE DE FR GB IT NL**

(56) Entgegenhaltungen:
**DE-A1-2 831 611**
**US-A-4 052 479**

(73) Patentinhaber: **BASF Aktiengesellschaft,**
**Carl-Bosch-Strasse 38, D-6700 Ludwigshafen (DE)**

(72) Erfinder: **Marosi, Laszlo, Dr., Londoner Ring 11,**
**D-6700 Ludwigshafen (DE)**
Erfinder: **Stabenow, Joachim, Dr., Am Feldrain 22,**
**D-6940 Weinheim (DE)**
Erfinder: **Schwarzmann, Matthias, Dr.,**
**Carl-Bosch-Strasse 54, D-6703 Limburgerhof (DE)**

Herstellung von Olefinen aus Rohmethanol

In neuerer Zeit gewinnen Bemühungen, Methanol zur Herstellung von Olefinen zu verwenden, zunehmendes Interesse. Methanol läßt sich aus Kohle, über Kohlevergasung und Herstellung von Synthesegas, mit Hilfe bewährter Technologien leicht herstellen. Gelingt es, Methanol in wirtschaftlicher Weise in niedere Olefine umzuwandeln, können die heute üblichen Weiterverarbeitungsverfahren der chemischen Industrie auch bei der Verwendung von Kohle als Rohstoff weiter beibehalten werden. In den vergangenen Jahren sind daher Verfahren entwickelt worden, die die Herstellung von Olefinen aus Methanol und/oder Dimethyläther zum Gegenstand haben. Ein solches Verfahren ist beispielsweise in der DE-OS 2 615 150 beschrieben. Als Katalysator wird dabei der Aluminosilikatzeolith ZSM-5 verwendet, der eigentlich ein Aromatisierungskatalysator ist. Die Umsetzung kann aber durch verschiedene Maßnahmen, insbesondere durch die Verkürzung der Verweilzeit, in Richtung Olefinbildung gelenkt werden. Weitere die Olefinbildung begünstigende Parameter sind insbesondere die Verdünnung von Methanol bzw. Dimethyläther mit Inertgasen oder die Verdünnung des Katalysators mit Bindemitteln. Die Erfahrung zeigt, daß hohe Olefinausbeuten nur durch eine sehr starke Verdünnung von Methanol und/oder Dimethyläther mit Inertgasen zu erzielen sind. Das Verfahren arbeitet daher nicht wirtschaftlich. Andere bekanntgewordene Verfahren haben als Nachteil eine geringe Belastbarkeit und eine schnelle Verkohlung des Katalysators. Es besteht daher großes Interesse an einem einfachen Verfahren, das die vollständige Umsetzung von Rohmethanol und/oder Dimethyläther in überwiegend aus $C_2-C_5$-Olefinen bestehende Kohlenwasserstoffe ermöglicht.

Es wurde nun gefunden, daß man in einfacher Weise $C_2$- bis $C_5$-Olefine aus Rohmethanol und/oder Dimethyläther durch katalytische Umwandlung bei erhöhter Temperatur in Gegenwart von zeolithhaltigen Katalysatoren erhält, wenn man als Katalysator einen eisenoxidhaltigen Zeolith verwendet.

Eine bevorzugte Ausführungsform besteht darin, Rohmethanol bei einem Druck zwischen Normaldruck und etwa 30 bar und bei Temperaturen zwischen 300 bis 700°C, bevorzugt bei 400 bis 650°C, an dem eisenoxidhaltigen Zeolithkatalysator umzusetzen. Unter Rohmethanol wird Methanol mit einem Wassergehalt bis etwa 30 Gew.-% verstanden, wie es in der Methanolsynthese entsteht. Dem Rohmethanol können auch noch andere niedere Alkohole beigemischt sein. Die Belastung des Katalysators, ausgedrückt in g Methanol und/oder Dimethyläther/g Kat · h, wird vorteilhafterweise so gewählt, daß diese Stoffe möglichst vollständig umgesetzt werden, damit keine Abtrenn- und Rückführprobleme entstehen. Im allgemeinen wird daher die Belastung in dem Bereich von 2 bis 50, vorzugsweise 5 bis 15 g/g Zeolith · h liegen. Es können jedoch ohne Nachteil für die Zusammensetzung des entstehenden Kohlenwasserstoffgemisches auch höhere Belastungen gewählt oder die Einsatzstoffe mit Inertgas, z. B. Stickstoff, verdünnt werden. Es ist aber ein besonderer Vorteil der Erfindung, daß man die Umsetzung von Rohmethanol bzw. Dimethyläther in $C_2$- bis $C_5$-Olefine ohne Verdünnungsmittel ausführen kann.

Als eisenoxidhaltige Zeolithkatalysatoren kann man die in der deutschen Patentanmeldung P 28 31 611.2 beschriebenen Zeolithe verwenden. Diese Katalysatoren zeichnen sich durch eine besonders hohe Aktivität und Selektivität, und darüber hinaus auch durch eine sehr hohe Laufzeit zwischen zwei Regenerationsstufen aus.

Die Durchführung des erfindungsgemäßen Verfahrens wird anhand der nachstehenden Beispiele näher erläutert.

### Beispiel

#### Herstellung des eisenoxidhaltigen Zeolithen

Es werden drei Lösungen hergestellt. Lösung 1 besteht aus 326,6 g technischem Wasserglas (8 Gew.-% $Na_2O$- und 28 Gew.-% $SiO_2$-Gehalt) und 352 g Wasser.

Lösung 2 besteht aus 300 g einer 50prozentigen wäßrigen Hexamethylendiaminlösung und Lösung 3 aus 508,3 g Wasser, 24,7 g 96prozentiger Schwefelsäure und 12,4 g $Fe_2(SO_4)_3 \cdot 18 H_2O$. Die Lösungen 2 und 3 werden nacheinander zu Lösung 1 unter Rühren hinzugegeben. Die erhaltene Mischung wird in einem Stahlautoklaven 5 Tage unter ihrem Eigendruck auf 150°C erhitzt. Das erhaltene Produkt wird filtriert, gewaschen und bei 100°C getrocknet.

#### Herstellung des Katalysators aus dem Zeolithen

Der erhaltene eisenoxidhaltige Zeolith wird in einem solchen Mengenverhältnis mit Böhmit vermischt, daß der Zeolithgehalt in der Mischung, bezogen auf Wasser und aminfreie Basis, etwa 65 Gew.-% beträgt. Die Mischung wird anschließend mit Wasser geknetet und zu Strängen mit 1 mm Durchmesser verstrangt. Die erhaltenen Stränge werden bei 540°C kalziniert und anschließend bei 80°C mit einer wäßrigen Ammoniumsulfatlösung behandelt, filtriert, gewaschen und getrocknet.

### Umsetzung von Rohmethanol zu Olefinen

20 g (Trockenbasis) dieses Katalysators werden in einen Durchflußreaktor mit 20 mm Durchmesser eingebaut und die Aktivität bei der Umsetzung von Rohmethanol in Olefine getestet. Die Reaktionsbedingungen und die Versuchsergebnisse sind in der folgenden Tabelle dargestellt. Die Reaktionsprodukte wurden gaschromatographisch analysiert.

| | |
|---|---|
| Eingangstemperatur | 380°C |
| Temperaturanstieg | 220°C |
| Druck | 1,4 bar |
| Belastung | 200 g Rohmethanol/h |
| Gesamtbelastung | 3000 g |
| Umsetzung | 95—100% |

Man erhält ein Reaktionsprodukt mit folgender Zusammensetzung:

Flüssige Kohlenwasserstoffe 15 Gew.-% bezogen auf eingesetztes $CH_2$

Gasförmige Kohlenwasserstoffe 85 Gew.-% bezogen auf eingesetztes $CH_2$

davon sind:

| | | |
|---|---|---|
| Olefine | $C_2$ | 10 Vol.-% |
| | $C_3$ | 50 Vol.-% |
| | $C_4$ | 20 Vol.-% |
| | $C_5$ | 6 Vol.-% |
| Paraffine | $C_1$ | 4 Vol.-% |
| | $C_2$ | <1 Vol.-% |
| | $C_3$ | 2 Vol.-% |
| | $C_4$ | 4 Vol.-% |
| | Rest | 3 Vol.-% |

### Patentansprüche

1. Verfahren zur Herstellung von $C_2$- bis $C_5$-Olefinen aus Rohmethanol und/oder Dimethyläther durch katalytische Umwandlung bei erhöhter Temperatur in Gegenwart von zeolithhaltigen Katalysatoren, dadurch gekennzeichnet, daß man als Katalysator einen eisenoxidhaltigen Zeolith verwendet.

2. Verfahren nach Anspruch 1, dadurch gekennzeichnet, daß man unverdünntes Rohmethanol und/oder Dimethyläther als Ausgangsstoff verwendet.

### Claims

1. A process for the preparation of $C_2-C_5$-olefins from crude methanol and/or dimethyl ether by catalytic conversion at an elevated temperature in the presence of a zeolitecontaining catalyst, characterized in that a zeolite containing iron oxide is used as catalyst.

2. A process as claimed in claim 1, characterized in that undiluted crude methanol and/or dimethyl ether is used as the starting material.

### Revendications

1. Procédé de préparation d'oléfines en $C_2$ à $C_5$ à partir de méthanol brut et(ou) d'éther diméthylique par réaction catalysée à température élevée en présence de catalyseurs à base d'une zéolithe, caractérisé en ce que l'on emploie comme catalyseur une zéolithe contenant de l'oxyde de fer.

2. Procédé suivant la revendication 1, caractérisé en ce que l'on emploie comme produit de départ du méthanol brut et(ou) de l'éther diméthylique non dilué.